# FASCICULE DE BREVET EUROPEEN

(11) **EP 1 369 435 B1**
(45) Date de publication et mention de la délivrance du brevet: **02.08.2006**
(21) Numéro de dépôt: 03292022.5
(22) Date de dépôt: 14.01.1999
(51) Int. Cl.: A61Q 5/00, A61K 8/06, A61K 8/81, A61Q 19/00, C08F 2/32

(54) **Latex épaississant, procédé de fabrication et applications en cosmétique**
Verdickend wirkendes Latex, Verfahren zu dessen Herstellung und Anwendungen in der Kosmetik
Thickening latex, manufacturing process and cosmetic applications

(30) Priorité: 16.01.1998 FR 9800464; 10.02.1998 FR 9801525; 04.08.1998 FR 9809999
(43) Date de publication de la demande: 10.12.2003
(62) Demande divisionnaire de: 99900919.4
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: Mallo Paul, 78400 Chatou (FR); Tabacchi Guy, 75007 Paris (FR); Boiteux Jean-Pierre, 81710 Saix (FR)
(74) Mandataire: Conan, Philippe Claude

(56) Documents cités:
- EP-A- 0 161 038
- EP-A- 0 793 957
- US-A- 4 906 701
- US-A- 5 185 395
- PATENT ABSTRACTS OF JAPAN vol. 1997, no. 10, 31 octobre 1997 (1997-10-31) & JP 9 157130 A (SHISEIDO CO LTD), 17 juin 1997 (1997-06-17)

## Description

La présente demande concerne des latex eau dans huile épaississants, leur procédé de préparation et leur application en tant qu'épaississant et/ou émulsionnant pour des produits de soins de la peau et des cheveux ou pour la fabrication de préparations cosmétiques, dermo-pharmaceutiques ou pharmaceutiques.

Différents épaississants existent et sont déjà utilisés pour ces usages. On connaît en particulier les produits naturels tels que les gommes de guar ou l'amidon mais dont les inconvénients sont ceux inhérents aux produits naturels, tels que la fluctuation des cours, les difficultés d'approvisionnement et une qualité aléatoire.

Les polymères synthétiques sous forme de poudre, principalement les polyacides acryliques sont également largement utilisés mais présentent l'inconvénient de nécessiter une neutralisation lors de l'utilisation, car ils ne développent leur viscosité qu'à partir d'un pH > 6.5 et leur mise en solution est souvent fastidieuse.

Il existe aussi des polymères épaississants synthétiques, se présentant sous forme de latex inverse, c'est-à-dire dont la phase continue est une huile. La mise en solution de ces latex est extrêmement rapide ; les polymères contenus dans ces latex inverses, sont le plus souvent des copolymères acrylamide / acrylate de métal alcalin ou acrylamide/acrylamido 2-méthyl 2-propane sulfonate de sodium ; ils sont déjà neutralisés et lorsqu'ils sont mis en solution dans l'eau, par exemple à une concentration de 1%, on observe que le pH est généralement supérieur à 6. On peut aussi citer les polymères décrits dans les brevets américains US 4,906,701 et US 5,185,395 qui sont des polymères linéaires utilisés dans l'industrie pétrolière.

Les copolymères acrylamide/acrylate de sodium ne développent cependant pas de propriétés épaississantes importantes lorsqu' on abaisse le pH en dessous de 6 ; par contre les copolymères acrylamide/acrylamido 2-méthyl 2-propane sulfonate de sodium décrits dans EP 0 503 853, gardent une capacité épaississante importante même à pH 4.

Cependant, de tels copolymères présentent des teneurs en monoacrylamide qui, bien qu'extrêmement faibles, pourraient conduire à rendre leur utilisation en cosmétique impossible dans un futur proche, suite à l'évolution de la législation européenne sur les substances dangereuses.

La demanderesse s'est donc intéressée à la synthèse et à la mise au point de polymères épaississants, même en pH acide, sous forme de latex inverse sans utiliser de mono-acrylamide.

L'invention a pour objet une composition comprenant une phase huile, une phase aqueuse, au moins un agent émulsifiant de type eau dans huile (E/H), au moins un agent émulsifiant de type huile dans eau (H/E), caractérisée en ce que ladite composition est un latex inverse comprenant de 20 % à 60 % en poids, et de préférence de 25 % à 45 % en poids, d'un polyélectrolyte anionique, branché ou réticulé, à base d'au moins un monomère possédant une fonction acide fort, copolymérisé avec au moins un monomère neutre à l'exception du mono-acrylamide et caractérisée en ce que 30 % à 90 % des motifs monomèriques que le polyélectrolyte anionique comprend, possèdent une fonction acide fort.

Par " agent émulsifiant du type eau dans huile ", on désigne des agents émulsifiants possédant une valeur de HLB suffisamment faible pour fournir des émulsions eau dans huile tels que les polymères tensioactifs commercialisés sous le nom de HYPERMER^{™} ou tels que les esters de sorbitan, comme le monooléate de sorbitan commercialisé par la Société SEPPIC sous le nom de marque MONTANE 80^{™}, ou l'isostéarate de sorbitan commercialisé par SEPPIC sous le nom de MONTANE 70^{™}.

Par " agent émulsifiant du type huile dans eau ", on désigne des agents émulsifiants possédant une valeur de HLB suffisamment élevée pour fournir des émulsions huile dans l'eau tels que les esters de sorbitan éthoxylés comme l'oléate de sorbitan éthoxylé avec 20 moles d'oxyde d'éthylène , commercialisé par la société SEPPIC sous le nom de MONTANOX^{™}80.

Par polymère branché, on désigne un polymère non linéaire qui possède des chaînes pendantes de manière à obtenir, lorsque ce polymère est mis en solution dans l'eau, un fort état d'enchevêtrement conduisant à des viscosités à bas gradient très importantes.

Par polymère réticulé, on désigne un polymère non linéaire se présentant à l'état de réseau tridimensionnel insoluble dans l'eau, mais gonflable à l'eau et conduisant donc à l'obtention d'un gel chimique.

La composition selon l'invention comporter des motifs réticulés et/ou des motifs branchés.

L'invention a notamment pour objet une composition telle que définie précédemment, caractérisée en ce que ledit polyélectrolyte anionique est le résultat d'une copolymérisation de ses monomères précurseurs effectuée à un pH inférieur à 4.

La fonction acide fort du monomère en comportant est notamment la fonction acide sulfonique ou la fonction acide phosphonique, partiellement ou totalement salifiée.

Ledit monomère peut être par exemple l'acide styrènesulfonique partiellement ou totalement salifié ; il est de préférence l'acide 2-méthyl 2-[(1-oxo 2-propènyl) amino] 1-propane-sulfonique partiellement ou totalement salifiée sous forme d'un sel de métal alcalin ou de sel d'ammonium.

Le monomère neutre est notamment choisi parmi l'acrylate de (2-hydroxy éthyle), l'acrylate de (2,3-dihydroxy propyle), le méthacrylate de (2-hydroxy éthyle), le méthacrylate de (2,3-dihydroxy propyle), ou un dérivé éthoxylé de poids moléculaire compris entre 400 et 1000, de chacun de ces esters.

Selon un aspect particulier de la présente invention, celle-ci a pour objet une composition comprenant une phase huile, une phase aqueuse, au moins un agent émulsifiant de type eau dans huile (E/H), au moins un agent émulsifiant de type huile dans eau (H/E), caractérisée en ce que ladite composition est un latex inverse comprenant de 20% à 60% en poids, et de préférence de 25% à 45% en poids, d'un polyélectrolyte anionique, branché ou réticulé, à base d'acide 2-méthyl 2-[(1-oxo 2-propènyl) amino] 1-propane sulfonique partiellement ou totalement salifié, copolymérisé avec de l'acrylate de (2-hydroxy éthyle) plus particulièrement, une composition telle que définie précédemment, caractérisée en ce que de 50% à 90%, en proportions molaires, des motifs monomériques que le poly-électrolyte anionique comprend, est l'acide 2-méthyl 2-[(1-oxo 2-propènyl) amino] 1-propanesulfonique (AMPS) partiellement ou totalement salifié sous forme d'un sel de métal alcalin ou de sel d'ammonium et notamment, une composition telle que définie précédemment, pour laquelle le polyélectrolyte anionique comporte, en proportions molaires, de 60% à 90% de sel de sodium ou de sel d'ammonium de l'acide 2-méthyl 2-[(1-oxo 2-propènyl) amino] 1-propane sulfonique et de 10% à 40% d'acrylate de (2-hydroxy éthyle).

L'invention a plus particulièrement pour objet une composition telle que définie précédemment, caractérisée en ce que le polyélectrolyte anionique est réticulé et/ou branché avec un composé diéthylènique ou polyéthylènique dans la proportion molaire exprimée par rapport aux monomères mis en oeuvre, de 0,005% à 1%, et de préférence de 0,01 % à 0,2 % et, plus particulièrement de 0,01 % à 0,1 %, de préférence celle pour laquelle l'agent de réticulation et/ou l'agent de ramification est choisi parmi le diméthacrylate d'éthylèneglycol, le diallyloxyacétate de sodium, le diacrylate d'éthylèneglycol, le diallyl urée, le triméthylol propanetriacrylate ou le méthylène-bis-(acrylamide).

Le latex selon l'invention contient généralement de 2,5% à 15% en poids, et de préférence de 4% à 9% en poids, d'agents émulsifiants, parmi lesquels de 20% à 50%, notamment de 25% à 40% du poids total des agents émulsifiants présents, sont du type eau dans huile (E/H) et dans laquelle de 80% à 50%, notamment de 75% à 60%, du poids total des agents émulsifiants, sont du type huile dans eau (H/E).

Selon un aspect particulier, la composition telle que définie précédemment, est caractérisée en ce que la phase huile représente de 15% à 40%, de préférence de 20% à 25%, de son poids total.

Cette phase huile est constituée soit par une huile minérale commerciale contenant des hydrocarbures saturés comme les paraffines, les isoparaffines, les cycloparaffines, présentant à température ambiante, une densité entre 0.7 et 0.9 et un point d'ébullition supérieur à 180°C, telle que par exemple l'EXXSOL^{™} D 100 S, ou le MARCOL^{™}52 commercialisés par EXXON CHEMICAL, l'isohexadécane ou l'isododécane, soit par une huile végétale, soit une huile de synthèse, soit par un mélange de plusieurs de ces huiles.

Selon un aspect préféré de la présente invention, la phase huile est constituée de MARCOL^{™} 52 ou d'isohexadécane ; l'isohexadécane, qui est identifié dans Chemical Abstracts par le numéro RN = 93685-80-4, est un mélange d'isoparaffines en C₁₂, C₁₆ et C₂₀ contenant au moins 97 % d'isoparaffines en C₁₆, parmi lesquelles le constituant principal est le 2,2,4,4,6,8,8-heptaméthyl nonane (RN = 4390-04-9). Il est commercialisé en France par la société BAYER. Le MARCOL^{™} 52 est une huile commerciale répondant à la définition des huiles de vaseline du Codex français. C'est une huile blanche minérale conforme aux réglementations FDA 21 CFR 172.878 et CFR 178.3620 (a) et elle est inscrite à la Pharmacopée des USA, US XXIII (1995) et à la Pharmacopée européenne (1993).

Les latex contiennent entre 20 % et 50 % d'eau. Les latex selon l'invention peuvent également contenir divers additifs tels que des agents complexants, des agents de transfert ou des agents limiteurs de chaîne.

Selon un autre aspect de la présente invention, celle-ci a pour objet un procédé de préparation de la composition telle que définie précédemment, caractérisé en ce que:
a) l'on émulsionne une solution aqueuse contenant les monomères et les éventuels additifs, dans une phase huile en présence d'un ou plusieurs agents émulsifiants de type eau dans huile,
b) l'on amorce la réaction de polymérisation par introduction dans l'émulsion formée en a), d'un initiateur de radicaux libres puis on la laisse se dérouler,
c) lorsque la réaction de polymérisation est terminée, on introduit un ou plusieurs agents émulsifiants de type huile dans eau à une température inférieure à 50°C.

Selon une variante de ce procédé, le milieu réactionnel issu de l'étape b), est concentré par distillation, avant la mise en oeuvre de l'étape c).

Selon une mise en oeuvre préférée du procédé tel que défini précédemment, la réaction de polymérisation est amorcée par un couple oxydo-réducteur, tel que le couple hydroperoxyde de cumène, métabisulfite de sodium, à une température inférieure ou égale à 10°C, puis conduite soit de manière quasi-adiabatique jusqu'à une température supérieure ou égale à 40°C, plus particulièrement supérieure ou égale à 50°C, soit en contrôlant l'évolution de la température.

Selon une autre mise en oeuvre préférée du procédé, la solution aqueuse de départ est ajustée à un pH inférieur ou égal à 4 avant la mise en oeuvre de l'étape a).

L'invention a aussi pour objet l'utilisation de la composition telle que définie précédemment pour préparer une composition topique cosmétique, dermo-pharmaceutique ou pharmaceutique.

Une composition topique selon l'invention, destinée à être appliquée sur la peau ou les muqueuses de l'homme ou de l'animal, peut consister en une émulsion topique comprenant au moins une phase aqueuse et au moins une phase huile. Cette émulsion topique peut être du type huile dans eau. Plus particulièrement, cette émulsion topique peut consister en une émulsion fluide, telle un lait ou un gel fluide. La phase huile de l'émulsion topique peut consister en un mélange d'une ou plusieurs huiles.

Une composition topique selon l'invention peut être destinée à une utilisation cosmétique ou être utilisée pour préparer un médicament destiné au traitement des maladies de la peau et des muqueuses. Dans ce dernier cas, la composition topique comporte alors un principe actif qui peut par exemple consister en un agent anti-inflammatoire, un myorelaxant, un antifongique ou un antibactérien.

Lorsque la composition topique est utilisée en tant que composition cosmétique destinée à être appliquée sur la peau ou les muqueuses, elle peut ou non comporter un principe actif, par exemple un agent hydratant, un agent bronzant, un filtre solaire, un antirides, un agent à visée amincissante, un agent antiradicalaire, un agent antiacnéique ou un antifongique.

Une composition topique selon l'invention comporte habituellement entre 0,1 % et 10 % en poids de l'agent épaississant défini ci-dessus. Le pH de la composition topique est de préférence supérieur ou égal à 5.

La composition topique peut en outre comporter des composés classiquement compris dans ce type de compositions, par exemple des parfums, des conservateurs, des colorants, des émollients ou des tensioactifs.

Selon encore un autre aspect, l'invention concerne l'utilisation du nouvel agent épaississant conforme à l'invention mentionné ci-dessus, pour épaissir et émulsionner une composition topique comprenant au moins une phase aqueuse.

La composition selon l'invention est un substitut intéressant à celles vendues sous le nom SEPIGEL^{™} 305 ou SEPIGEL^{™} 501 par la demanderesse, car elle présente aussi une bonne compatibilité avec les autres excipients utilisés pour la préparation de formulations telles que les laits, les lotions, les gels, les crèmes, les gels crèmes, les savons, les bains moussants, les baumes, les shampooings ou les après-shampooings. Elle peut aussi être mise en oeuvre avec lesdits SEPIGEL.

Elle est notamment compatible avec les concentrés décrits et revendiqués dans les publications internationales WO 92/06778, WO 95/04592, WO 95/13863, WO 96/37285, WO 98/22207, WO 98/47610 ou dans FR 2734 496, avec les agents tensioactifs décrits dans WO 93/08204.

Elle est particulièrement compatible avec le MONTANOV® 68, le MONTANOV^{™} 82, le MONTANOV^{™} 202 ou le SEPIPERL^{™} N. Elle peut également être utilisée dans des émulsions du type de celles décrites et revendiquées dans EP 0 629 396 et dans les dispersions aqueuses cosmétiquement ou physiologiquement acceptable avec un composé organo polysiloxane choisi, par exemple parmi ceux décrits dans WO 93/05762 ou dans WO 93/21316.

Elle peut également être utilisée pour former des gels aqueux à pH acide cosmétiquement ou physiologiquement acceptables , tels que ceux décrit dans WO 93/07856; elle peut également être utilisée en association avec des celluloses non-ioniques, pour former par exemple des gels de coiffage tels que ceux décrits dans EP 0 684 024, ou encore en association avec des esters d'acides gras et de sucre , pour former des compositions pour le traitement du cheveux ou de la peau telles que celles décrites dans EP 0 603 019. ou encore dans les shampooings ou après-shampooings tels que décrits et revendiqués dans WO 92/21316 ou enfin en association avec un homopolymère anionique tels que le CARBOPOL^{™} pour former des produits de traitement des cheveux comme ceux décrits dans DE 195 23596.

La composition selon l'invention est également compatible avec les principe actifs tels que par exemple, les agents auto-bronzants comme le dihydroxy-acétone (DHA) ou les agents anti-acné; elle peut donc être introduite dans des compositions auto-bronzantes comme celles revendiquées dans EP 0 715 845, EP 0 604249, EP 0576188 ou dans WO 93/07902.

Elle est également compatible avec les dérivés N-acylés d'aminoacides, ce qui permet son utilisation dans des compositions apaisantes notamment pour peau sensible, telles que celles décrites ou revendiquées dans WO 92/21318, WO 94/27561, ou WO 98/09611.

Les exemples qui suivent ont pour but d'illustrer la présente invention.

### Exemple 1: Préparation et propriétés du latex inverse selon l'invention

### A] Préparation

**a)** On charge dans un bécher, sous agitation :
- 608 ,8 g d'une solution commerciale à 50% du sel de sodium de l'acide 2-methyl-2[(1-oxo-2 propènyl) amino] 1-propanesulfonique,
- 72,6 g d'acrylate de (2-hydroxy éthyle),
- 0,18 g de diéthylène triaminepentacétate de sodium, et
- 0,121 g de méthylène bis(acrylamide,)

Le pH de la phase aqueuse précédemment décrit est ajusté à 3,5, par ajout de 0,7g d'acide 2-methyl-2[(1-oxo-2 propènyl) amino] 1-propane-sulfonique.

Parallèlement, on prépare une phase organique en introduisant dans un bécher agité successivement :
- 220 g d'iso hexadécane,
- 25 g de Montane 80 VG (oléate de sorbitan éthoxylé à 20 moles d'oxyde d'éthylène, commercialisé par SEPPIC) et
- 0,2 g d'azo bis(isobutyronitrile).

La phase aqueuse est introduite progressivement dans la phase organique puis soumise à une agitation mécanique violente de type ultra-turrax^{™} commercialisé par IKA.

L'émulsion obtenue, caractérisée par une viscosité à 25°C de 2600mPas (Brookfield RVT, Mobile 4, vitesse 20), est alors transférée dans un réacteur de polymérisation. L'émulsion est soumise à un barbotage d'azote important de manière à éliminer l'oxygène et refroidit à environ 5-6°C.

On introduit alors 10 g d'une solution à 1,1% en poids de matière active d'hydroperoxyde de cumène dans l'iso hexadécane. Après un temps suffisant pour une bonne homogénéisation de la solution, on introduit en environ 25 minutes, 25 g d'une solution aqueuse de métabisulfite de sodium (solution à 0,2%). Pendant cette introduction, on laisse monter la température dans le réacteur de polymérisation jusqu'à la température finale de polymérisation puis on maintient le milieu réactionnel pendant environ 90 minutes à cette température. L'ensemble est ensuite refroidi jusqu'à une température d'environ 35°C et on introduit lentement 50 g de MONTANOX ^{™} 80 VG .

On obtient l'émulsion désirée.

### Evaluation des propriétés :

- Viscosité à 20°C du latex à 3% dans l'eau
   (Brookfield RVT, Mobile 6, vitesse 20): η = 36700 mPas ; le pH est de 5,1.

On abaisse le pH à 3,7 et l'on obtient alors le résultat suivant: η = 31000 mPas

On constate que le produit final est exempt de mono-acrylamide.

**b)** En opérant de la même manière qu'au paragraphe a) en abaissant la quantité de méthylène bis(acrylamide) de 0,121 g à 0,091g, on obtient une émulsion ayant les caractéristiques de viscosité suivantes :
- Viscosité à 20°C du latex à 3% dans l'eau
   (Brookfield RVT Mobile 6, vitesse 20): η = 33000 mPas ; le pH est de 5,2. Après abaissement du pH, on obtient les résultats suivants :
   à pH = 4,0, η = 31000 mPas ;
   à pH = 2,8, η = 18300 mPas.

On constate que le produit final est exempt de mono-acrylamide.

c) En opérant de la même manière qu'au paragraphe a) en abaissant la quantité de méthylène bis(acrylamide) de 0,121 g à 0,084g, celle d'acrylate de (2-hydroxy éthyle) de 72,6 g à 53g, et en augmentant la quantité de solution commerciale à 50% du sel de sodium de l'acide 2-methyl-2[(1-oxo-2 propènyl) amino] 1-propane sulfonique de 608,8g à 628g, on obtient une émulsion ayant les caractéristiques de viscosité suivantes :
- Viscosité à 20°C du latex à 3% dans l'eau (Brookfield RVT Mobile 6, vitesse 20): η = 27400 mPas ; le pH est de 5,2.

Après abaissement du pH, on obtient les résultats suivants :
à pH = 4,0, η = 27400 mPas ;
à pH = 2,8, η = 18200 mPas .

On constate que le produit final est exempt de mono-acrylamide.

On constate que le toucher des émulsions obtenues est très particulier à partir de 1% de polymère dans la solution et que cette différence s'accentue avec l'augmentation de la concentration ; il s'agit d'un toucher très frais au début qui fond complètement sur la peau, toucher que l'on ne ressent pas du tout avec les latex de l'état de la technique.

Les exemples suivants mettent en oeuvre indifféremment les émulsions préparées selon l'un des paragraphes A]a), A]b) et A]c) (appelés dans les exemples suivants - composé de l'exemple 1).

### B] Propriétés

On prépare avec chacun des latex préparés aux paragraphes A]a), A]b) et A]c), des formules cosmétiques comprenant :
0,5%, 1%, 1,5%, 2%, 2,5% ou 3% de latex
5% de SIMULSOL 165,
20% de LANOL 1688
0,5% de SEPICIDE HB
eau qsp 100%.

On constate que le toucher des émulsions obtenues, est très particulier à partir de 1% de polymère dans la solution et que cette différence s'accentue avec l'augmentation de la concentration ; il s'agit d'un toucher très frais au début, qui fond complètement sur la peau, toucher que l'on ne ressent pas du tout avec les latex de l'état de la technique.

### Exemple 2 : Crème de soin

| | |
|---|---|
| Cyclométhicone : | 10% |
| Composé de l'exemple 1 : | 0,8 % |
| MONTANOV^{™} 68 : | 4,5 % |
| Conservateur : | 0,65 % |
| Lysine : | 0,025 % |
| EDTA (sel disodique) : | 0,05 % |
| Gomme de xanthane : | 0,2 % |
| Glycérine : | 3% |
| Eau : | qsp 100 % |

### Exemple 3 : Crème de soin

| | |
|---|---|
| Cyclométhicone : | 10 % |
| Composé de l'exemple 1 : | 0,8 % |
| MONTANOV^{™} 68 : | 4,5 % |
| Perfluoropolymethylisopropylether : | 0,5 % |
| Conservateur : | 0,65 % |
| Lysine : | 0,025 % |
| EDTA (sel disodique) : | 0,05 % |
| PEMULEN^{™}TR: | 0,2 % |
| Glycérine : | 3 % |
| Eau : | qsp 100 % |

### Exemple 4 : Baume après-rasage

### FORMULE

| | |
|---|---|
| A Composé de l'exemple 1 : | 1,5 % |
| Eau : | q.s.p 100 % |
| B MICROPEARL^{™} M 100: | 5,0% |
| SEPICIDE^{™} CI: | 0,50 % |
| Parfum: | 0,20 % |
| Ethanol 95°: | 10,0 % |

### MODE OPERATOIRE

Ajouter B dans A.

### Exemple 5: Emulsion satinée pour le corps

### FORMULE

| | | |
|---|---|---|
| A | SIMULSOL^{™} 165 : | 5,0 % |
| | LANOL^{™} 1688 : | 8,50 % |
| | Beurre de Karité : | 2 % |
| | Huile de paraffine : | 6,5 % |
| | LANOL ^{™} 14M : | 3 % |
| | LANOL^{™} S : | 0,6 % |
| B | Eau : | 66,2 % |
| C | MICROPEARL^{™} M 100: | 5 % |
| D | Composé de l'exemple 1: | 3 % |
| E | SEPICIDE^{™} CI : | 0,3 % |
| | SEPICIDE^{™} HB : | 0,5 % |
| | MONTEINE^{™} CA : | 1 % |
| | Parfum : | 0,20 % |
| | Acétate de vitamine E : | 0,20 % |
| | Sodium pyrolidinone carboxylate: | 1 % (agent hydratant) |

### MODE OPERATOIRE

Ajouter C dans B, émulsionner B dans A à 70°C, puis ajouter D à 60°C puis E à 30°C.

### Exemple 6: Lait corporel

### FORMULE

| | | |
|---|---|---|
| A | SIMULSOL^{™} 165: | 5,0 % |
| | LANOL^{™} 1688: | 12,0 % |
| | LANOL^{™} 14M: | 2,0 % |
| | Alcool cétylique: | 0,3 % |
| | SCHERCEMOL^{™} OP: | 3 % |
| B | Eau : | q.s.p. 100 % |
| C | Composé de l'exemple 1 : | 0,35 % |
| D | SEPICIDE ^{™} CI : | 0,2 % |
| | SEPICIDE^{™} HB : | 0,5 % |
| | Parfum : | 0,20 % |

| | | |
|---|---|---|
| (Le SCHERCEMOL^{™} OP est un ester émollient à effet non gras) | | |

### MODE OPERATOIRE

Emulsionner B dans A vers 75°C ; ajouter C vers 60°C, puis D vers 30°C

### Exemple 7 : crème H/E

### FORMULE

| | | |
|---|---|---|
| A | SIMULSOL^{™} 165: | 5,0 % |
| | LANOL^{™} 1688: | 20,0 % |
| | (additif à effet stabilisant) | 1,0 % |
| B | Eau : | q.s.p. 100 % |
| C | Composé de l'exemple 1 : | 2,50 % |
| D | SEPICIDE^{™} CI : | 0,20 % |
| | SEPICIDE^{™} HB : | 0,30 % |

### MODE OPERATOIRE

Introduire B dans A vers 75°C ; ajouter C vers 60°C, puis D vers 45°C

### Exemple 8 : gel solaire non gras

### FORMULE

| | | |
|---|---|---|
| A | Composé de l'exemple 1 : | 3,00 % |
| | Eau : | 30 % |
| B | SEPICIDE^{™} C : | 0,20 % |
| | SEPICIDE^{™} HB : | 0,30 % |
| | Parfum: | 0,10 % |
| C | Colorant : | q.s. |
| | Eau: | 30 % |
| D | MICROPEARL^{™} M 100 : | 3,00% |
| | Eau : | q.s.p 100 % |
| E | Huile de silicone : | 2,0 % |
| | PARSOL^{™} MCX : | 5,00 % |

### MODE OPERATOIRE

Introduire B dans A; ajouter C,puis D, puis E.

### Exemple 9 : Lait solaire

### FORMULE

| | | |
|---|---|---|
| A | SEPIPERL^{™} N : | 3,0 % |
| | Huile de sésame : | 5,0 % |
| | PARSOL^{™} MCX : | 5,0 % |
| | Carraghénane λ : | 0,10 % |
| B | Eau : | q.s.p. 100 % |
| C | Composé de l'exemple 1 : | 0,80 % |
| D | Parfum: | q.s. |
| | Conservateur : | q.s. |

### MODE OPERATOIRE

Emulsionner B dans A à 75°C puis ajouter C vers 60°C, puis D vers 30°C et ajuster le pH si nécessaire

### Exemple 10 : Gel de massage

### FORMULE

| | | |
|---|---|---|
| A | Composé de l'exemple 1: | 3,5 % |
| | Eau: | 20,0 % |
| B | Colorant : | 2 gouttes/100g |
| | Eau: | q.s. |
| C | Alcool: | 10 % |
| | Menthol : | 0,10 % |
| D | Huile de silicone : | 5,0 % |

### MODE OPERATOIRE

Ajouter B dans A; puis ajouter au mélange, C puis D

### Exemple 11 : gel soin de massage

### FORMULE

| | | |
|---|---|---|
| A | Composé de l'exemple 1 : | 3,00 % |
| | Eau: | 30 % |
| B | SEPICIDE^{™} CI : | 0,20 % |
| | SEPICIDE^{™} HB: | 0,30 % |
| | Parfum: | 0,05 % |
| C | colorant : | q.s. |
| | Eau: | q.s.p 100 % |
| D | MICROPEARL^{™} SQL: | 5,0 % |
| | LANOL^{™} 1688 : | 2 % |

### MODE OPERATOIRE

Préparer A; additionner B, puis C, puis D.

### Exemple 12 : Gel coup d'éclat

### FORMULE

| | | |
|---|---|---|
| A | Composé de l'exemple 1: | 4 % |
| | Eau : | 30 % |
| B | ELASTINE HPM: | 5,0 % |
| C | MICROPEARL^{™} M 100: | 3 % |
| | Eau : | 5% |
| D | SEPICIDE^{™} CI: | 0,2 % |
| | SEPICIDE^{™} HB: | 0,3 % |
| | Parfum: | 0,06 % |
| | Sodium pyrolidinone carboxylate 50 %: | 1 % |
| | Eau : | q.s.p. 100 % |

### MODE OPERATOIRE

Préparer A; additionner B, puis C, puis D.

### Exemple 13: Lait corporel

### FORMULE

| | | |
|---|---|---|
| A | SEPIPERL^{™}N: | 3,0 % |
| | Triheptonate de glycerol : | 10,0 % |
| B | Eau : | q.s.p. 100 % |
| C | Composé de l'exemple 1: | 1,0 % |
| D | Parfum : | q.s. |
| | Conservateur : | q.s. |

### MODE OPERATOIRE

Fondre A à environ 75°C. émulsionner B dans A à 75°C, ajouter C vers 60°C, puis D.

### Exemple 14 : Emulsion démaquillante à l'huile d'amande douce

### FORMULE

| | |
|---|---|
| MONTANOV^{™}68: | 5 % |
| Huile d'amandes douces : | 5 % |
| Eau : | q.s.p. 100 % |
| Composé de l'exemple 1: | 0,3 % |
| Glycérine : | 5 % |
| Conservateur : | 0,2 % |
| Parfum : | 0,3 % |

### Exemple 15 : Crème hydratante pour peaux grasses

### FORMULE

| | |
|---|---|
| MONTANOV^{™} 68: | 5 % |
| Cétylstéaryloctanoate : | 8 % |
| Octyl palmitate : | 2 % |
| Eau: | q.s.p. 100 % |
| Composé de l'exemple 1: | 0,6 % |
| MICROPEARL^{™} M100: | 3,0 % |
| Mucopolysaccharides: | 5 % |
| SEPICIDE^{™} HB: | 0,8 % |
| Parfum: | 0,3 % |

### Exemple 16 : Baume après-rasage apaisant sans alcool

### FORMULE

| | |
|---|---|
| Mélange de lauryl aminoacides : | 0,1 % à 5 % |
| Aspartate de magnésium et de potassium : | 0,002 % à 0,5 % |
| LANOL^{™} 99 : | 2 % |
| Huile d'amandes douces : | 0,5 % |
| Eau : | q.s.p. 100 % |
| Composé de l'exemple 1 : | 3 % |
| SEPICIDE^{™} HB : | 0,3 % |
| SEPICIDE^{™} CI : | 0,2 % |
| Parfum : | 0,4 % |

### Exemple 17 : Crème aux AHA pour peaux sensibles

### FORMULE

| | |
|---|---|
| Mélange de lauryl aminoacides : | 0,1 % à 5 % |
| Aspartate de magnésium et de potassium : | 0,002 % à 0,5 % |
| LANOL^{™} 99 : | 2 % |
| MONTANOV^{™}68: | 5,0 % |
| Eau : | q.s.p. 100 % |
| Composé de l'exemple 1 : | 1,50 % |
| Acide gluconique: | 1,50 % |
| Triéthanolamine : | 0,9 % |
| SEPICIDE^{™} HB : | 0,3 % |
| SEPICIDE^{™}CI : | 0,2 % |
| Parfum : | 0,4 % |

### Exemple 18 : Soin apaisant après-soleil

### FORMULE

| | |
|---|---|
| Mélange de lauryl aminoacides: | 0,1 % à 5 % |
| Aspartate de magnésium et de potassium: | 0,002 % à 0,5 % |
| LANOL^{™} 99: | 10,0 % |
| Eau: | q.s.p. 100 % |
| Composé de l'exemple 1: | 2,50 % |
| SEPICIDE^{™} HB: | 0,3 % |
| SEPICIDE^{™} CI: | 0,2 % |
| Parfum: | 0,4 % |
| Colorant: | 0,03 % |

### Exemple 19 : Lait démaquillant

### FORMULE

| | |
|---|---|
| SEPIPERL^{™}N | 3 % |
| PRIMOL 352: | 8,0 % |
| Huile d'amandes douces: | 2 % |
| Eau: | q.s.p 100 % |
| Composé de l'exemple 1: | 0,8 % |
| Conservateur: | 0,2 % |

### Exemple 20: Lait corporel

### FORMULE

| | |
|---|---|
| SEPIPERL^{™}N: | 3,5 % |
| LANOL^{™} 37T: | 8,0 % |
| SOLAGUM^{™}L: | 0,05 % |
| Eau: | q.s.p 100 % |
| Benzophénone: | 2,0 % |
| Diméthicone 350cPs: | 0,05 % |
| Composé de l'exemple 1: | 0,8 % |
| Conservateur: | 0,2 % |
| Parfum: | 0,4 % |

### Exemple 21: émulsion fluide à pH alcalin

| | |
|---|---|
| MARCOL^{™} 82: | 5,0 % |
| NaOH: | 10,0 % |
| Eau: | q.s.p. 100 % |
| Composé de l'exemple 1: | 1,5 % |

### Exemple 22 : Fond de teint fluide

### FORMULE

| | |
|---|---|
| SIMULSOL^{™} 165 | 5,0 % |
| LANOL^{™} 84D: | 8,0 % |
| LANOL^{™} 99: | 5,0 % |
| Eau: | q.s.p. 100 % |
| Pigments et charges minérales: | 10,0 % |
| Composé de l'exemple 1: | 1,2 % |
| Conservateur: | 0,2 % |
| Parfum: | 0,4 % |

### Exemple 23 : Lait solaire

### FORMULE

| | |
|---|---|
| SEPIPERL^{™} N | 3,5% |
| LANOL^{™} 37T: | 10,0 % |
| PARSOL NOX^{™}: | 5,0 % |
| EUSOLEX^{™} 4360: | 2,0 % |
| Eau: | q.s.p. 100 % |
| Composé de l'exemple 1: | 1,8 % |
| Conservateur: | 0,2 % |
| Parfum: | 0,4% |

### Exemple 24 : Gel contour des yeux

### FORMULE

| | |
|---|---|
| Composé de l'exemple 1: | 2,0 % |
| Parfum: | 0,06 % |
| Sodium pyrrolidinonecarboxylate: | 0,2 % |
| DOW CORNING^{™} 245 FLuid | 2,0 % |
| Eau: | q.s.p. 100 % |

### Exemple 25: composition de soin non rincée

### FORMULE

| | |
|---|---|
| Composé de l'exemple 1: | 1,5 % |
| Parfum: | q.s |
| Conservateur: | q.s. |
| DOW CORNING^{™} X2 8360: | 5,0 % |
| DOW CORNING^{™} Q2 1401: | 15 % |
| Eau: | q.s.p. 100 % |

### Exemple 26: gel amincissant

| | |
|---|---|
| Composé de l'exemple 1 | 5 % |
| Ethanol | 30 % |
| Menthol | 0,1 % |
| Caféine | 2,5 % |
| Extrait de ruscus | 2 % |
| Extrait de lierre | 2% |
| SEPICIDE^{™} HB | 1 % |
| Eau | q.s.p. 100 % |

### Exemple 27 : Baume après-rasage apaisant sans alcool

### FORMULE

| | | |
|---|---|---|
| A | LIPACIDE^{™} PVB : | 1,0 % |
| | LANOL^{™} 99 : | 2,0 % |
| | Huile d'amandes douces : | 0,5 % |
| B | Composé de l'exemple 1 | 3,5 % |
| C | Eau: | q.s.p. 100 % |
| D | Parfum: | 0,4 % |
| | SEPICIDE^{™} HB : | 0,4 % |
| | SEPICIDE^{™} CI : | 0,2 % |

### Exemple 28: Gel rafraîchissant après-rasage

### FORMULE

| | | |
|---|---|---|
| A | LIPACIDE^{™} PVB : | 0,5 % |
| | LANOL^{™} 99 : | 5,0 % |
| | Composé de l'exemple 1 | 2,5 % |
| B | eau : | q.s.p 100 % |
| C | MICROPEARL^{™} LM : | 0,5% |
| | Parfum : | 0,2 % |
| | SEPICIDE^{™} HB : | 0,3 % |
| | SEPICIDE^{™} CI : | 0,2 % |

### Exemple 29: Soin pour les peaux grasses

### FORMULE

| | | |
|---|---|---|
| A | MICROPEARL^{™}M310: | 1,0 % |
| | Composé de l'exemple 1 | 5,0 % |
| | Isononanoate d'octyle : | 4.0 % |
| B | Eau : | q.s.p 100 % |
| C | SEPICONTROL^{™} A5 : | 4,0 % |
| | Parfum : | 0,1 % |
| | SEPICIDE^{™} HB : | 0,3 % |
| | SEPICIDE^{™} CI : | 0,2 % |
| D | CAPIGEL^{™} 98 : | 0,5 % |
| | Eau: | 10% |

### Exemple 30 : Crème aux AHA

### FORMULE

| | | |
|---|---|---|
| A | MONTANOV^{™} 68 : | 5,0 % |
| | LIPACIDE^{™} PVB: | 1,05 % |
| | LANOL^{™} 99 : | 10,0 % |
| B : | Eau | q.s.p. 100 % |
| | Acide gluconique : | 1,5 % |
| | TEA (triéthanolamine) : | 0,9 % |
| C | Composé de l'exemple 1 | 1,5 % |
| D | Parfum: | 0,4 % |
| | SEPICIDE^{™} HB: | 0,2 % |
| | SEPICIDE^{™} CI: | 0,4 % |

### Exemple 31 : Autobronzant non gras pour visage et corps

### FORMULE

| | | |
|---|---|---|
| A | LANOL^{™} 2681 : | 3,0 % |
| | Composé de l'exemple 1 | 2,5 % |
| B | Eau : | q.s.p. 100 % |
| | Dihydroxyacétone : | 3,0 % |
| C | Parfum : | 0,2 % |
| | SEPICIDE^{™} HB : | 0,8 % |
| | NaOH (hydroxyde de sodium) : | qs pH = 5 |

### Exemple 32 : Lait solaire au monoï de Tahiti

### FORMULE

| | | |
|---|---|---|
| A | Monoï de Tahiti: | 10 % |
| | LIPACIDE^{™} PVB : | 0,5 % |
| | Composé de l'exemple 1 | 2,2 % |
| B | Eau : | q.s.p. 100 % |
| C | Parfum: | 0,1 % |
| | SEPICIDE HB : | 0,3 % |
| | SEPICIDE^{™} CI : | 0,1 % |
| | Méthoxycinnamate d'octyle : | 4,0 % |

### Exemple 33 : Soin solaire pour le visage

### FORMULE

| | | |
|---|---|---|
| A | Cyclométhicone et diméthiconol : | 4,0 % |
| | Composé de l'exemple 1 | 3,5 % |
| B Eau : | | q.s.p. 100 % |
| C | Parfum: | 0,1 % |
| | SEPICIDE^{™} HB : | 0,3 % |
| | SEPICIDE^{™} CI : | 0,21 % |
| | Méthoxycinnamate d'octyle : | 5,0 % |
| | Micatitane : | 2,0 % |
| | Acide lactique : | q.s.p. pH = 6,5 |

### Exemple 34 : Emulsion bronzante sans soleil

### FORMULE

| | | |
|---|---|---|
| A | LANOL^{™} 99 : | 15 % |
| | MONTANOV^{™} 68 : | 5,0 % |
| | Paraméthoxycinnamate d'octyle : | 3,0 % |
| B | Eau : | q.s.p. 100 % |
| | Dihydroxyacétone : | 5,0 % |
| | Phosphate monosodique : | 0,2 % |
| C | Composé de l'exemple 1 | 0,5 % |
| D | Parfum : | 0,3% |
| | SEPICIDE^{™} HB : | 0,8% |
| | NaOH : | q.s. pH=5. |

### Exemple 35 : Gel brillance

| | |
|---|---|
| Composé de l'exemple 1 | 1,5 % |
| Silicone volatil | 25 % |
| Monopropylèneglycol | 25 % |
| Eau déminéralisée | 10 % |
| Glycérine | qsp 100 % |

### Exemple 36 : Gel amincissant

| | |
|---|---|
| Composé de l'exemple 1 | 1,5 % |
| Isononylisononanoate | 2 % |
| Caféine | 5 % |
| Ethanol | 40 % |
| MICROPEARL™ LM | 2 % |
| Eau déminéralisée | q.s.p. 100 % |
| Conservateur parfum | q.s. |

### Exemple 37 : Lait démaquillant

| | |
|---|---|
| SIMULSOL™ 165 | 4 % |
| MONTANOV^{™} 202 | 1 % |
| Caprylate-caprate triglyceride | 15 % |
| PECOSIL^{™} DCT | 1 % |
| Eau déminéralisée | qs |
| CAPIGEL^{™} 98 | 0,5 % |
| Composé de l'exemple 1: | 1 % |
| PROTEOL^{™} OAT | 2 % |
| NaOH | q.s.p. pH 7 |

### Exemple 38 : Crème solaire

| | |
|---|---|
| SIMULSOL^{™} 165 | 3 % |
| MONTANOV^{™} 202 | 2 % |
| Benzoate C12-C15 | 8 % |
| PECOSIL^{™} PS 100 | 2 % |
| Diméthicone | 2 % |
| Cyclométhicone | 5 % |
| Octyl-méthoxy-cinnamate | 6 % |
| Benzophénone-3 | 4 % |
| Oxyde de Titane | 8 % |
| Gomme xanthane | 0,2 % |
| Butylène-glycol | 5 % |
| Eau déminéralisée | q.s.p. 100 % |
| Composé de l'exemple 1 | 1,5 % |
| Conservateur, parfum | qs |

### Exemple 39 : Gel de soin peaux mixtes

| | |
|---|---|
| Composé de l'exemple 1 | 4 % |
| Squalane végétal | 5 % |
| Dimethicone | 1,5 % |
| SEPICONTROL^{™} A5 | 4 % |
| Gomme xanthane | 0,3 % |
| Eau | q.s.p. 100 % |
| Conservateur, Parfum | qs |

### Exemple 40 : Voile parfumé pour le corps

| | |
|---|---|
| Composé de l'exemple 1 | 1,5 % |
| Cyclométhicone | 5% |
| Parfum | 2 % |
| MICROPEARL^{™} M100 | 5 % |
| Glycérine | 5 % |
| Eau déminéralisée | qsp 100 % |

### Exemple 41 : Crème vitaminée

| | |
|---|---|
| SIMULSOL^{™} 165 | 5 % |
| MONTANOV^{™} 202 | 1 % |
| Caprylic/capric triglycerides | 20 % |
| Palmitate de vitamine A | 0,2 % |
| Acetate de vitamine E | 1 % |
| MICROPEARL^{™} M305 | 1,5 % |
| Composé de l'exemple 1 | 0,7 % |
| Eau | q.s.p. 100 % |
| Conservateur, parfum | qs |

Le MONTANOV^{™} 68 (cétéaryl glucoside), est une composition auto-émulsionnable telle que décrite dans WO 92/06778, commercialisée par la société SEPPIC.

Le MICROPEARL^{™} M 100 est une poudre ultra fine au toucher très doux et à action matifiante commercialisée par la société MATSUMO

Le SEPICIDE^{™} CI, imidazolidine urée, est un agent conservateur commercialisé par la société SEPPIC.

PEMULEN^{™} TR est un polymère acrylique commercialisé par GOODRICH.

Le SIMULSOL^{™} 165 est du stéarate de glycérol auto-émulsionnable commercialisée par la société SEPPIC.

Le LANOL^{™} 1688 est un ester émollient à effet non gras commercialisé par la société SEPPIC.

Le LANOL^{™} 14M et le LANOL® S sont des facteurs de consistance commercialisés par la société SEPPIC.

Le SEPICIDE^{™} HB, qui est un mélange de phénoxyéthanol, de méthyl paraben, d'éthylparaben, de propylparaben et de butylparaben, est un agent conservateur commercialisé par la société SEPPIC.

Le MONTEINE^{™} CA est un agent hydratant commercialisé par la société SEPPIC.

Le SCHERCEMOL^{™} OP est un ester émollient à effet non gras.

Le LANOL^{™} P est un additif à effet stabilisant commercialisé par la société SEPPIC.

Le PARSOL^{™} MCX est de l'octyl paraméthoxycinnamate; commercialisé par la société GIVAUDAN.

Le SEPIPERL^{™} N est un agent nacrant, commercialisé par la société SEPPIC, à base d'un mélange d'alkyl poly glucosides tels que ceux décrits dans WO 95/13863.

Le MICROPEARL^{™} SQL est un mélange de micro particules renfermant du squalane qui se libère sous l'action du massage; il est commercialisé par la société MATSUMO.

Le LANOL^{™} 99 est de l'isononyl isononanoate commercialisé par la société SEPPIC.

Le LANOL^{™} 37T est du triheptanoate de glycérol, commercialisé par la société SEPPIC.

Le SOLAGUM^{™} L est un carraghénane commercialisé par la société SEPPIC.

Le MARCOL^{™} 82 est une huile de paraffine commercialisée par la société EXXON.

Le LANOL^{™} 84D est du malate de dioctyle commercialisé par la société SEPPIC.

Le PARSOL NOX^{™} est un filtre solaire commercialisé par la société GIVAUDAN.

l'EUSOLEX^{™} 4360 est un filtre solaire commercialisé par la société MERCK.

Le DOW CORNING^{™} 245 Fluid est de la cyclométhicone, commercialisée par la société DOW CORNING.

Le LIPACIDE^{™} PVB, est un hydrolysat de protéines de blé acylé commercialisé par la société SEPPIC.

Le MICROPEARL^{™} LM est un mélange de squalane, de polyméthylméthacrylate et de menthol, commercialisé par la société SEPPIC.

Le SEPICONTROL^{™} A5 est un mélange capryloy glycine, sarcosine, extrait de cinnamon zylanicum, commercialisé par la société SEPPIC, tel que ceux décrits dans la demande internationale de brevet PCT/FR98/01313 déposée le 23 juin 1998 (WO 99/00109).

Le CAPIGEL^{™} 98 est un copolymère acrylique commercialisé par la société SEPPIC.

Le LANOL^{™} 2681 est un mélange caprylate, caprate de coprah, commercialisé par la société SEPPIC.

Le MONTANOV^{™} 202, est une composition APG/alcools gras telle que décrite dans WO 98/47610, commercialisée par la société SEPPIC.

## Revendications

1. Composition comprenant une phase huile, une phase aqueuse, au moins un agent émulsifiant de type eau dans huile (E/H), au moins un agent émulsifiant de type huile dans eau (H/E), **caractérisée en ce que** ladite composition est un latex inverse comprenant de 20% à 60% en poids, et de préférence de 25% à 45% en poids, d'un polyélectrolyte anionique, branché ou réticulé, à base d'au moins un monomère possédant une fonction acide fort, copolymérisé avec au moins un monomère neutre, à l'exception du mono-acrylamide et **caractérisée en ce que** 30 % à 90 % des motifs monomèriques que le polyélectrolyte anionique comprend, possèdent une fonction acide fort.

2. Composition telle que définie à la revendication 1, **caractérisée en ce que** ledit polyélectrolyte anionique est le résultat d'une copolymérisation de ses monomères précurseurs effectuée à un pH inférieur à 4.

3. Composition telle que définie à l'une des revendications 1 ou 2, pour laquelle la fonction acide fort du monomère en comportant, est la fonction acide sulfonique ou la fonction acide phosphonique, partiellement ou totalement salifié et de préférence, ledit monomère est l'acide 2-méthyl 2-[(1-oxo 2-propènyl) amino] 1-propane-sulfonique partiellement ou totalement salifiée sous forme d'un sel de métal alcalin ou de sel d'ammonium.

4. Composition telle que définie à l'une des revendications 1 à 3, pour laquelle le monomère neutre est choisi parmi l'acrylate de (2-hydroxy éthyle), l'acrylate de (2,3-dihydroxy propyle), le méthacrylate de (2-hydroxy éthyle), le méthacrylate de (2,3-dihydroxy propyle), ou un dérivé éthoxylé, de poids moléculaire compris entre 400 et 1000 de chacun de ces esters.

5. Composition telle que définie à l'une des revendications 1 à 4, comprenant une phase huile, une phase aqueuse, au moins un agent émulsifiant de type eau dans huile (E/H), au moins un agent émulsifiant de type huile dans eau (H/E), **caractérisée en ce que** ladite composition est un latex inverse comprenant de 20% à 60% en poids, et de préférence de 25% à 45% en poids, d'un polyélectrolyte anionique, branché ou réticulé, à base d'acide 2-méthyl 2-[(1-oxo 2-propènyl) amino] 1-propanesulfonique partiellement ou totalement salifié, co-polymérisé avec de l'acrylate de (2-hydroxy éthyle).

6. Composition telle que définie à l'une des revendications 1 à 5, **caractérisée en ce que** 30% à 90%, de préférence 50% à 90% en proportions molaires, des motifs monomériques que le polyélectrolyte anionique comprend, est l'acide 2-méthyl 2-[(1-oxo 2-propènyl) amino] 1-propanesulfonique partiellement ou totalement salifié sous forme d'un sel de métal alcalin ou de sel d'ammonium, notamment une composition telle que définie précédemment, pour laquelle le polyélectrolyte anionique comporte, en proportions molaires, de 60% à 90% de sel de sodium ou de sel d'ammonium de l'acide 2-méthyl 2-[(1-oxo 2-propènyl) amino] 1-propanesulfonique et de 10% à 40% d'acrylate de (2-hydroxy éthyle).

7. Composition telle que définie à l'une quelconque des revendications 1 à 6, **caractérisé en ce que** le polyélectolyte anionique est réticulé et/ou branché avec un composé diéthylènique ou polyéthylènique dans la proportion molaire exprimée par rapport aux monomères mis en oeuvre, de 0,005% à 1%, et de préférence de 0,01% à 0,2 % et plus particulièrement de 0,01 % à 0,1 %.

8. Composition telle que définie à la revendication 7, pour laquelle l'agent de réticulation et/ou l'agent de ramification est choisi parmi le diméthacrylate d'éthylèneglycol, le diallyloxyacétate de sodium, le diacrylate d'éthylèneglycol, le diallyl urée, le triméthylol propanetriacrylate ou le méthylène-bis-(acrylamide).

9. Composition telle que définie à l'une quelconque des revendications 1 à 8, **caractérisée en ce qu'**elle contient de 2,5% à 15% en poids, et de préférence de 4% à 9% en poids, d'agents émulsifiants.

10. Composition telle que définie à la revendication 9, dans laquelle de 20% à 50%, notamment de 25% à 40% du poids total des agents émulsifiants présents sont du type eau dans huile (E/H) et dans laquelle de 80% à 50%, notamment de 75% à 60%, du poids total des agents émulsifiants, sont du type huile dans eau (H/E).

11. Composition telle que définie à l'une quelconque des revendications 1 à 10, **caractérisée en ce que** la phase huile représente de 15% à 40%, de préférence de 20% à 25%, de son poids total.

12. Composition telle que définie à la revendication 11 dans laquelle la phase huile est constituée d'isohexadécane ou d'huile blanche minérale.

13. Composition telle que définie à l'une quelconque des revendications 1 à 12, **caractérisée en ce qu'**elle contient en outre un ou plusieurs additifs choisis notamment parmi les agents complexants, les agents de transfert ou les agents limiteurs de chaînes.

14. Procédé de préparation de la composition telle que définie à l'une des revendications 1 à 13, **caractérisé en ce que**
a) l'on émulsionne une solution aqueuse contenant les monomères et les éventuels additifs, dans une phase huile en présence d'un ou plusieurs agents émulsifiants de type eau dans huile,
b) l'on amorce la réaction de polymérisation par introduction dans l'émulsion formée en a), d'un initiateur de radicaux libres puis on la laisse se dérouler,
c) lorsque la réaction de polymérisation est terminée, l'on introduit un ou plusieurs agents émulsifiants de type huile dans eau à une température inférieure à 50°C.

15. Procédé tel que défini à la revendication 14, selon laquelle le milieu réactionnel issu de l'étape b), est concentré par distillation, avant la mise en oeuvre de l'étape c).

16. Procédé tel que défini à l'une des revendications 14 ou 15, dans lequel la réaction de polymérisation est amorcée par un couple oxydo-réducteur, tel que le couple hydroperoxyde de cumène -disulfite de sodium, à une température inférieure ou égale à 10°C, puis conduite soit de manière quasi-adiabatique jusqu'à une température supérieure ou égale à 40°C, plus particulièrement supérieure ou égale à 50°C, soit en contrôlant l'évolution de la température.

17. Procédé tel que défini à l'une des revendications 14 à 16, dans lequel la solution aqueuse de départ est ajustée à un pH inférieur ou égal à 4 avant la mise en oeuvre de l'étape a).

18. Utilisation de la composition telle que définie à l'une des revendications 1 à 13, pour préparer une composition topique cosmétique, dermo-pharmaceutique ou pharmaceutique.

19. Composition topique cosmétique, dermo-pharmaceutique ou pharmaceutique comprenant de 0,1 % à 10 % en poids d'un latex inverse tel que défini à l'une des revendications 1 à 13.

20. Composition telle que définie à la revendication 19, sous la forme d'un lait, d'une lotion, d'un gel, d'une crème, d'un gel crème, d'un savon, d'un bain moussant, d'un baume, d'un shampooing ou d'un après-shampooing.

21. Composition telle que définie à l'une des revendications 19 ou 20, pour apaiser les peaux sensibles.

## Claims

1. Composition comprising an oil phase, an aqueous phase, at least one emulsifier of water-in-oil (W/O) type, at least one emulsifier of oil-in-water (O/W) type, **characterized in that** the said composition is an inverted latex comprising from 20% to 60% by weight, and preferably from 25% to 45% by weight, of a branched or crosslinked anionic polyelectrolyte based on at least one monomer possessing a strongly acidic function, copolymerized either with at least one neutral monomer, with the exception of monoacrylamide, and **characterized in that** 30% to 90% of the monomer units which comprise the anionic polyelectrolyte have a strongly acidic function.

2. Composition as defined in Claim 1, **characterized in that** the said anionic polyelectrolyte is the result of a copolymerization of its precursor monomers, which is carried out at a pH below 4.

3. Composition as defined in either of Claims and 2, for which the strongly acidic function of the monomer containing it is a sulphonic acid function or a phosphonic acid function, partially or totally salified and preferably, the said monomer is 2-methyl-2-[(1-oxo-2-propenyl)amino]-1-propanesulphonic acid, partially or totally salified in the form of an alkali metal salt or an ammonium salt.

4. Composition as defined in one of Claims 1 to 3, for which the neutral monomer is chosen from 2-hydroxyethyl acrylate, 2,3-dihydroxypropyl acrylate, 2-hydroxyethyl methacrylate and 2,3-dihydroxypropyl methacrylate, or an ethoxylated derivative, with a molecular weight of between 400 and 1000, of each of these esters.

5. Composition as defined in one of Claims 1 to 4, comprising an oil phase, an aqueous phase, at least one emulsifier of water-in-oil (W/O) type and at least one emulsifier of oil-in-water (O/W) type, **characterized in that** the said composition is an inverted latex comprising from 20% to 60% by weight, and preferably from 25% to 45% by weight, of a branched or crosslinked, anionic polyelectrolyte based on partially or totally salified 2-methyl-2-[(1-oxo-2-propenyl)amino]-1-propanesulfonic acid, copolymerized with 2-hydroxyethyl acrylate.

6. Composition as defined in one of Claims 1 to 5, **characterized in that** 30% to 90%, preferably 50% to 90%, in molar proportions, of the monomer units comprised by the anionic polyelectrolyte is 2-methyl-2-[(1-oxo-2-propenyl)amino]-1-propanesulfonic acid partially or totally salified in the form of an alkali metal salt or an ammonium salt, in particular a composition as defined above, for which the anionic polyelectrolyte includes, in molar proportions, from 60% to 90% of sodium or of ammonium salt of 2-methyl-2-[(1-oxo-2-propenyl)amino]-1-propanesulfonic acid and from 10% to 40% of 2-hydroxyethyl acrylate.

7. Composition as defined in any one of Claims 1 to 6, **characterized in that** the anionic polyelectrolyte is crosslinked and/or branched with a diethylenic or polyethylenic compound in a molar proportion, expressed relative to the monomers used, of from 0.005% to 1% and preferably from 0.01% to 0.2% and more particularly from 0.001 to 0.1%.

8. Composition as defined in Claim 7, for which the crosslinking agent and/or the branching agent is chosen from ethylene glycol dimethacrylate, sodium diallyloxyacetate, ethylene glycol diacrylate, diallylurea, trimethylolpropane triacrylate or methylenebisacrylamide.

9. Composition as defined in any one of Claims 1 to 8, **characterized in that** it contains from 2.5% to 15% by weight, and preferably from 4% to 9% by weight, of emulsifiers.

10. Composition as defined in Claim 9, in which from 20% to 50%, in particular from 25% to 40%, of the total weight of the emulsifiers present are of the water-in-oil (W/O) type and in which from 80% to 50%, in particular from 75% to 60%, of the total weight of the emulsifiers are of the oil-in-water (O/W) type.

11. Composition as defined in any one of Claims 1 to 10, **characterized in that** the oil phase represents from 15% to 40%, preferably from 20% to 25%, of its total weight.

12. Composition as defined in Claim 11 in which the oily phase is made up of isohexadecane or mineral white oil.

13. Composition as defined in any one of Claims 1 to 12, **characterized in that** it also contains one or more additives chosen in particular from complexing agents, transfer agents or chain-limiting agents.

14. Process for preparing the composition as defined in one of Claims 1 to 13, **characterized in that**
a) an aqueous solution containing the monomers and the optional additives is emulsified in an oil phase in the presence of one or more emulsifiers of water-in-oil type,
b) the polymerization reaction is initiated by introducing a free-radical initiator into the emulsion formed in a), after which the reaction is left to proceed,
c) when the polymerization reaction is complete, one or more emulsifiers of oil-in-water type are introduced at a temperature below 50°C.

15. Process as defined in Claim 14, according to which the reaction medium obtained after step b) is concentrated by distillation before step c) is carried out.

16. Process as defined in either of Claims 14 and 15, in which the polymerization reaction is initiated by a redox couple, such as the cumene hydroperoxide/sodium disulphite couple, at a temperature below or equal to 10°C, and is then carried out, either in a virtually adiabatic manner up to a temperature above or equal to 40°C, more particularly above or equal to 50°C, or by controlling the change in the temperature.

17. Process as defined in one of Claims 14 to 16, in which the starting aqueous solution is adjusted to a pH below or equal to 4 before step a) is carried out.

18. Use of the composition as defined in one of Claims 1 to 13, for preparing a cosmetic, dermo-pharmaceutical or pharmaceutical topical composition.

19. Cosmetic, dermo-pharmaceutical or pharmaceutical topical composition comprising from 0.1% to 10% by weight of an inverted latex as defined in one of Claims 1 to 13.

20. Composition as defined in Claim 19, in the form of a milk, a lotion, a gel, a cream, a cream-gel a soap, a foam bath, a balm, a shampoo or a conditioner.

21. Composition as defined in either of Claims 19 and 20, for soothing sensitive skin.

## Patentansprüche

1. Zusammensetzung, enthaltend eine Ölphase, eine wäßrige Phase, mindestens einen Emulgator vom Wasser-in-Öl-Typ (W/O-Typ) und mindestens einen Emulgator vom Öl-in-Wasser-Typ (O/W-Typ), **dadurch gekennzeichnet, daß** es sich bei der Zusammensetzung um einen inversen Latex mit 20 bis 60 Gew.-% und vorzugsweise 25 bis 45 Gew.-% eines verzweigten oder vernetzten anionischen Polyelektrolyts auf Basis mindestens eines Monomers mit einer stark sauren Funktion, das mit mindestens einem neutralen Monomer mit Ausnahme von Monoacrylamid copolymerisiert ist, handelt und 30 bis 90 Gew.-% der Monomereinheiten, die der anionische Polyelektrolyt enthält, eine stark saure Funktion aufweisen.

2. Zusammensetzung nach Anspruch 1, **dadurch gekennzeichnet, daß** es sich bei dem anionischen Polyelektrolyt um das Ergebnis einer Copolymerisation der Vorläufermonomere dafür bei einem pH-Wert von weniger als 4 handelt.

3. Zusammensetzung nach Anspruch 1 oder 2, für die es sich bei der stark sauren Funktion des diese enthaltenden Monomers um eine teilweise oder vollständig in die Salzform überführte Sulfonsäurefunktion oder eine Phosphonsäurefunktion und vorzugsweise um teilweise oder vollständig in die Alkalimetallsalz- oder Ammoniumsalzform überführte 2-Methyl-2-[(1-oxo-2-propenyl)amino]-1-propansulfonsäure handelt.

4. Zusammensetzung nach einem der Ansprüche 1 bis 3, für die das neutrale Monomer unter 2-Hydroxyethylacrylat, 2,3-Dihydroxypropylacrylat, 2-Hydroxyethylmethacrylat, 2,3-Dihydroxypropylmethacrylat oder einem ethoxylierten Derivat ausgewählt ist, wobei diese Ester jeweils ein Molekulargewicht zwischen 400 und 1000 aufweisen.

5. Zusammensetzung nach einem der Ansprüche 1 bis 4, enthaltend eine Ölphase, eine wäßrige Phase, mindestens einen Emulgator vom Wasser-in-Öl-Typ (W/O-Typ) und mindestens einen Emulgator vom Öl-in-Wasser-Typ (O/W-Typ), **dadurch gekennzeichnet, daß** es sich bei der Zusammensetzung um einen inversen Latex mit 20 bis 60 Gew.-% und vorzugsweise 25 bis 45 Gew.-% eines verzweigten oder vernetzten anionischen Polyelektrolyts auf Basis von teilweise oder vollständig in die Salzform überführter 2-Methyl-2-[(1-oxo-2-propenyl)amino]-1-propansulfonsäure, die mit 2-Hydroxyethylacrylat copolymerisiert ist, handelt.

6. Zusammensetzung nach einem der Ansprüche 1 bis 5, **dadurch gekennzeichnet, daß** 30 bis 90 Mol-% und vorzugsweise 50 bis 90 Mol-% der Monomereinheiten, die der anionische Polyelektrolyt enthält, teilweise oder vollständig in die Alkalimetallsalz- oder Ammoniumsalzform überführte 2-Methyl-2-[(1-oxo-2-propenyl)amino]-1-propansulfonsäure sind, insbesondere eine Zusammensetzung gemäß obiger Definition, für die der anionische Polyelektrolyt 60 bis 90 Mol-% Natriumsalz von 2-Methyl-2-[(1-oxo-2-propenyl)amino]-1-propansulfonsäure oder Ammoniumsalz von 2-Methyl-2-[(1-oxo-2-propenyl)amino]-1-propansulfonsäure und 10 bis 40 Mol-% 2-Hydroxyethylacrylat enthält.

7. Zusammensetzung nach einem der Ansprüche 1 bis 6, **dadurch gekennzeichnet, daß** der anionische Polyelektrolyt mit einer diethylenischen oder polyethylenischen Verbindung in einem molaren Anteil, bezogen auf die eingesetzten Monomere, von 0,005 bis 1%, vorzugsweise 0,01 bis 0,2% und insbesondere 0,01 bis 0,1% vernetzt und/oder verzweigt ist.

8. Zusammensetzung nach Anspruch 7, für die das Vernetzungsmittel und/oder Verzweigungsmittel unter Ethylenglykoldimethacrylat, Natriumdiallyloxyacetat, Ethylenglykoldiacrylat, Diallylharnstoff, Trimethylolpropantriacrylat und Methylenbis(acrylamid) ausgewählt ist bzw. sind.

9. Zusammensetzung nach einem der Ansprüche 1 bis 8, **dadurch gekennzeichnet, daß** sie 2,5 bis 15 Gew.-% und vorzugsweise 4 bis 9 Gew.-% Emulgatoren enthält.

10. Zusammensetzung nach Anspruch 9, in der 20 bis 50% und insbesondere 25 bis 40% des Gesamtgewichts der vorliegenden Emulgatoren vom Wasser-in-Öl-Typ (W/O-Typ) und 80 bis 50% und insbesondere 75 bis 60% des Gesamtgewichts der vorliegenden Emulgatoren vom Öl-in-Wasser-Typ (O/W-Typ) sind.

11. Zusammensetzung nach einem der Ansprüche 1 bis 10, **dadurch gekennzeichnet, daß** die Ölphase 15 bis 40% und vorzugsweise 20 bis 25% ihres Gesamtgewichts ausmacht.

12. Zusammensetzung nach Anspruch 11, in der die Ölphase aus Isohexadecan oder Weißöl besteht.

13. Zusammensetzung nach einem der Ansprüche 1 bis 12, **dadurch gekennzeichnet, daß** sie außerdem auch noch ein oder mehrere Additive, die insbesondere unter Komplexbildnern, Kettenübertragungsmitteln oder Kettenreglern ausgewählt sind, enthält.

14. Verfahren zur Herstellung der Zusammensetzung nach einem der Ansprüche 1 bis 13, **dadurch gekennzeichnet, daß** man
a) eine die Monomere und die fakultativen Additive enthaltende wäßrige Lösung in Gegenwart eines oder mehrerer Emulgatoren vom wasser-in-Ö1-Typ in einer Ölphase emulgiert,
b) die Polymerisationsreaktion durch Eintragen eines radikalbildenden Initiators in die in a) gebildete Emulsion initiiert und dann ablaufen läßt und
c) nach Beendigung der Polymerisation bei einer Temperatur von weniger als 50°C einen oder mehrere Emulgatoren vom Öl-in-Wasser-Typ einträgt.

15. Verfahren nach Anspruch 14, bei dem man die Reaktionsmischung aus Schritt b) vor der Durchführung von Schritt c) destillativ aufkonzentriert.

16. Verfahren nach Anspruch 14 oder 15, bei dem man die Polymerisationsreaktion mit einem Redoxpaar, wie dem Paar Cumolhydroperoxid/Natriumdisulfit, bei einer Temperatur kleiner gleich 10°C initiiert und dann entweder quasi-adiabatisch bis zu einer Temperatur größer gleich 40°C, insbesondere größer gleich 50°C, oder unter Steuerung der Temperaturentwicklung fährt.

17. Verfahren nach einem der Ansprüche 14 bis 16, bei dem man die als Ausgangsmaterial dienende wäßrige Lösung vor der Durchführung von Schritt a) auf einen pH-Wert kleiner gleich 4 einstellt.

18. Verwendung einer Zusammensetzung nach einem der Ansprüche 1 bis 13 zur Herstellung einer topischen kosmetischen, dermopharmazeutischen oder pharmazeutischen Zusammensetzung.

19. Topische kosmetische, dermopharmazeutische oder pharmazeutische Zusammensetzung, enthaltend 0,1 bis 10 Gew.-% eines inversen Latex nach einem der Ansprüche 1 bis 13.

20. Zusammensetzung nach Anspruch 19 in Form einer Milch, einer Lotion, eines Gels, einer Creme, eines Cremegels, einer Seife, eines Schaumbads, eines Balsams, eines Shampoos oder einer Haarpflegespülung.

21. Zusammensetzung nach Anspruch 19 oder 20 zur Pflege von empfindlicher Haut.
